**Europäisches Patentamt**

**European Patent Office**

**Office européen des brevets**

(19)

(11) Publication number: **0 084 419**
**B1**

(12) # EUROPEAN PATENT SPECIFICATION

(45) Date of publication of patent specification: **02.09.87**

(51) Int. Cl.⁴: **A 61 B 17/38,** A 61 B 1/30

(21) Application number: **83300105.0**

(22) Date of filing: **10.01.83**

(54) Resectoscopes.

(30) Priority: **14.01.82 JP 3506/82 u**

(43) Date of publication of application:
**27.07.83 Bulletin 83/30**

(45) Publication of the grant of the patent:
**02.09.87 Bulletin 87/36**

(84) Designated Contracting States:
**AT BE CH DE FR GB IT LI NL SE**

(56) References cited:
**FR-A-2 400 351**
**GB-A- 626 182**
**US-A-2 487 502**

(73) Proprietor: **OLYMPUS OPTICAL CO., LTD.**
**43-2, 2-chome, Hatagaya Shibuya-ku**
**Tokyo 151 (JP)**

(72) Inventor: **Kubota, Tetsumaru**
**Olympus Oowada ryo No. 22-13, Oowadamachi**
**4-chome**
**Hachioji-shi Tokyo (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

Courier Press, Leamington Spa, England.

## Description

This invention relates to resectoscopes such as the type designed to be inserted into a bladder through the urethra, and having an electrode projectable at its tip, via which a high frequency electric current can be passed to perform a resection on an affected part whilst it is being observed through the resectoscope.

Such resectoscopes comprise an optical observation system, an electrode, a resectoscope insertion sheath, in which the optical system and electrode are inserted, a grip provided at the rear part of this resectoscope insertion sheath, and a movable member securing the electrode at the rear end, and provided with a finger trigger such that the fingers of one hand can engage the grip of the resectoscope insertion sheath and the finger trigger of the movable member to grip them and cause one to slide within the other to urge the movable member forward or rearward, to make the electrode at the tip advance or retract from an opening at the tip of the resectoscope insertion sheath to make a resection or the like.

In known resectoscopes of this type, the affected part resecting electrode is electrically connected at the rear to an electrode lead by forming an electrode inserting hole from the tip of the movable member to a rear middle position, and providing on the respective side surfaces of the movable member in the region containing this inserting hole a mouthpiece inserting hole, which includes a plug inserting hole in which a plug at the tip of the electrode lead is inserted, and a separate pressing member inserting hole diametrically opposite the electrode inserting hole, and the rear end of the electrode inserted in the electrode inserting hole is pressed on a mouthpiece inserted through the mouthpiece inserting hole in the opposite position on the tip surface of a pressing member formed to be comparatively small and inserted through the above mentioned pressing member inserting hole, so that the electrode may be pressed at the rear end on the mouthpiece and the high frequency current fed from the current source may be passed to the above mentioned electrode through the electrode lead, the plug at the tip of the electrode lead and the mouthpiece having the inserting hole in which this plug is inserted.

With such a structure for connecting the electrode with the mouthpiece, the electrode is pressed at the rear end into contact with the mouthpiece with a pressing force applied at the tip of a small diameter pressing member, and therefore there have been problems in that, in order that the electrode may be well connected with the mouthpiece, not only the pressing member and the mouthpiece with the electrode held between them must be aligned with each other in the producing process, so as to be correctly opposed to each other but also, unless they are aligned with each other with high precision, the electrical contact of the electrode with the mouthpiece will fail.

Also, if the pressing force applied to the pressing member is very large, or the repeated use in a given time, in which the pressing force to the pressing member is alternately released and applied too frequently, then the surface of contact of the tip of the pressing member with the rear end of the electrode or the surface of contact of the electrode at the rear end with the mouthpiece may be worn or damaged and, as a result, the electrical contact may fail and little or no high frequency current will be fed to the electrode, so that it will not be possible to effect an efficient resection on an affected part.

Further, there is suggested in U.S. Patent No 2 487 502 a prior art construction wherein a sheathed electrode is held in a slit clamping plate at the rear end, and a tightening screw is arranged in a direction at right angles to the above mentioned slit so as to pull a holding piece on the side beyond the above mentioned slit toward the hand side, to hold the sheathed electrode adjacent the rear end within the slit, and to electrically connect the end of the electrode to an electrical contact within an insulating hollow mouthpiece behind the clamping plate.

However, in such a prior art construction there have been problems in that, as a telescope viewing system needs to be inserted through the mouthpiece in an insulated manner, and in such a way that the telescope and electrode can be rotatably fitted and the telescope arranged in the centre, the tightening screw has to be arranged toward one side of the body, and the structure for accommodating this tightening screw and fitting it to the body will be complicated, and the production cost will be high.

Further, there is a danger that, as the mouthpiece part is exposed, the operator may accidentally touch the mouthpiece part and suffer an electric shock.

There have been further problems, as moisture and dust may be deposited on the outer surface, so that the insulation will not be sufficient, and even if the device is adequately insulated for direct currents, the high frequency dielectric loss may become large and the insulation insufficient, and when current is passed there will be a danger of electric shock, so that the safety margin will be low.

An object of the present invention is to provide an embodiment in the form of a resectoscope wherein an electrode at the rear end is positively electrically connected to an electrode lead at the tip in a simple manner, yet with high safety, and reduced risk of being broken or damaged in use.

According to one exemplary embodiment of the invention there is provided a resectoscope comprising an optical viewing system, an axially movable current-carrying electrode, an insertion sheath within which the viewing system and electrode are inserted, an axially movable member fixing the electrode at its rear end for controlling its axial movement, said movable member comprising an electrically insulating portion provided with an axial bore, an electrode fixing member mounted to the movable member for fixing the rear end of the electrode, said electrode fixing member comprising a slit to form a flexible piece and an electrode holding space within this slit, a clamping member for radially pressing the flexible piece to narrow the width of the electrode holding space, and a plug connecting part to which a plug at the tip of an electrode lead can be connected for electrically connecting the electrode and electrode lead together, said clamping member and plug connecting part being fitted to the lateral surface of the movable member, characterised in that the movable member is made of an electrically insulating material, in that the electrode fixing member is fitted in said axial bore, the clamping member and the plug connecting member being fitted to different side faces of the movable member, and in that the plug connecting part is in direct electrical contact with the electrode fixing member.

The invention will now be described with reference to the drawings, in which:—

Figure 1 is a side view of a first exemplary embodiment of the invention;

Figure 2 is a cross-sectional view on the line A—A of Figure 1;

Figure 3 is a partly sectional view on the line B—B of Figure 2;

Figure 4 is a cross-sectional view of a second exemplary embodiment of the invention;

Figure 5 is a partly sectional view on the line C—C of Figure 4; and

Figure 6 is a cross-sectional view of a third exemplary embodiment of the invention.

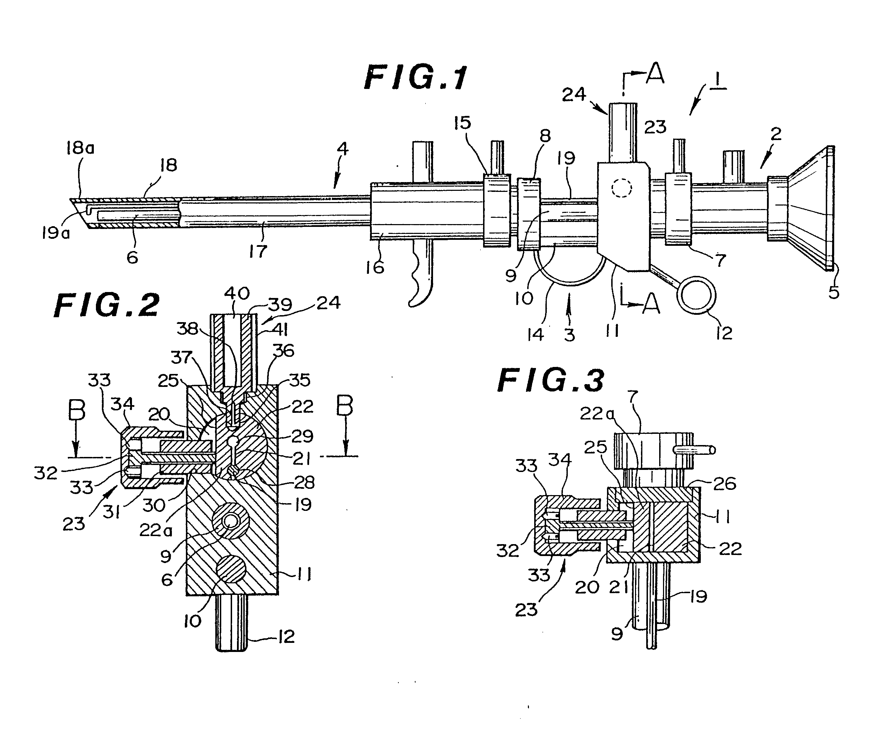

In the embodiment illustrated in Figures 1 to 3, a resectoscope 1 is formed by an optical viewing system 2, a resectoscope handle 3 to which the viewing system 2 is removably fitted, and a resectoscope insertion sheath 4 removably fitted to the resectoscope handle 3. The viewing system 2 comprises an eyepiece 5 and an elongate tubular part 6. The viewing system 2 and the resectoscope handle 3 are removably connected together by a connecting part 7 provided on the resectoscope handle 3. The resectoscope handle 3 connects the connecting part 7 to a forward fixed member 8 via an intermediate guide pipe 9 and intermediate guide shaft 10. A movable member 11 is slidably fitted to the guide pipe 9 and guide shaft 10. A finger trigger 12 is fitted to the movable member 11. The movable member 11 and fixed member 8 are connected together by a plate spring 14 in this exemplary embodiment. The resectoscope handle 3 and resectoscope insertion sheath 4 are removably connected together by a connecting part 15 provided on the resectoscope insertion sheath 4. The resectoscope insertion sheath 4 comprises the connecting part 15 for engaging the resectoscope handle 3, a resectoscope sheath body 16, and a resectoscope insertion sheath tube 17. The resectoscope insertion sheath tube 17 is formed by an outer tube 18 of metal, such as brass or stainless steel, and an insulated tip portion 18a, formed of insulating material, such as an epoxy resin or ceramic, and fitted on the tip of the outer tube 18. The elongate tubular part 6 of the viewing system is inserted through the guide pipe 9 of the resectoscope handle 3 into an internal path of the outer tube 18. A current-conducting electrode 19 is inserted to extend parallel with the elongate tubular part 6. This electrode 19 has, for example, a loop-shaped electrode tip 19a provided to project at the tip so as to be able to resect an affected part (prostate) when heated by the passage of an electric current therethrough.

This electrode 19 is supported and secured at the rear end by an electrode fixing structure, shown in Figure 2, to be electrically connected to an external electrode lead supplying electric current to the electrode 19.

The electrode fixing structure is formed of a resilient electrode fixing member 22 arranged within a space 20 formed by partly boring the interior of the movable member 11, which is formed of a highly insulating material such as polytetrafluroethylene (PTFE) to hold the electrode 19 at the rear end between a flexible piece 22a and the main body of the fixing member, the flexible piece being formed by providing a slit 21, and a clamping member 23 pressing a pressed surface 25 of the flexible piece 22a to narrow the width of the slit 21, and an electrode lead connecting part 24 electrically connected at the tip to the above mentioned electrode fixing member 22.

The space 20 in the movable member 11 is in the form of a circular bore and an opening is provided for inserting the electrode fixing member 22 through a rear end plate 26 of the movable member 11, as shown in Figure 3, and is so formed that its axial direction may be parallel with the above mentioned electrode 19.

The electrode fixing member 22 is a conductive member in the form of a column of the same shape as, but slightly shorter than the space 20, is closed at the rear by the end plate 26 formed of insulating material such as PTFE. The slit 21 is formed in this electrode fixing member 22. The rectangular pressed surface 25 is formed on a side face of the electrode fixing member 22 by cutting off a segment at one side. The flexible piece 22a is formed by the residual thin portion between this pressed surface 25 and slit 21.

The slit 21 opens on the lower side surface of the electrode fixing member 22, and is formed to the requisite depth in the electrode fixing member 22, parallel with the pressed surface 25 formed from the front face to the rear face of the electrode fixing member 22. An electrode holding space 28 of a circular section, having substantially the same diameter as the diameter of the rear end of the current-carrying electrode 19 is formed in a position near the outer periphery at which this slit 21 opens. At the deepest part of this slit 21, an expanded bore 29 is provided to give resilience to the flexible piece 22a.

The clamping member 23 pressing against the pressed surface 25 is fitted in an opening (through hole) 30 formed on the side face of the movable member 11 so as to face the pressed surface 25 of the electrode fixing member 22. This clamping member 23 is formed by a guide tube 31 fitted securely in the opening 30 of the movable member 11, with its tip near to the pressed surface 25, and with a female screw-thread formed on its inner surface, a press rod 32 screwed into this guide tube 31 to advance and retract inwardly and outwardly as guided by the guide tube 31, so as to contact the pressed surface 25 of the flexible piece 22a and bear against it with the requisite pressure, and a control grip 34 of insulating material fixed to the rear end of the press-rod 32 by screws 33. The movable member 11 and guide tube 31 may be integrally formed, instead of using the separate parts mentioned above. When the control grip 34 is rotated, the press-rod 32 will advance into the space 20 and press against the surface 25 of the flexible piece 22a to narrow the width of the slit 21. When the press-rod 32 is retracted, the width of the above mentioned slit 21 will be expanded by the resilience of the flexible piece 22a.

The electrode lead connecting part 24 is formed by an engaging hole 35 of appropriate depth formed on the upper face of the electrode fixing member 22, opposite the slit 21, as shown in the drawings (Figures 1 and 2) of this embodiment, a stepped, expanding fitting opening 36 provided in the upper outer face of the movable member 11 to coaxially communicate with and expose this engaging hole 35, a connecting mouthpiece 39 inserted in this fitting opening 36 and pressed at the tip into the above mentioned engaging hole 35, a plug inserting hole 40 provided inward from the upper end face of this connecting mouthpiece 39 to insert the plug at the tip of the electrode lead, and an insulating member fitted around this connecting mouthpiece 39, pressed at the lower tip into the movable member 11 and made flush with the upper face of the connecting mouthpiece 39 at the upper end.

The connecting mouthpiece 39 is formed at the tip of a small diameter, and has a slit 38 formed in this tip part so as to be able to be pressed into the above mentioned engaging hole 35. Thus, the electrode fixing member 22 arranged within the movable member 11 is fixed to the movable member 11 by the connecting mouthpiece 39 engaged through the movable member 11 from the outside so as to be prevented from moving forward or rearward or rotating.

The thus formed first embodiment is characterised in that, in the resectoscope comprising the viewing system, current-carrying electrode, resectoscope insertion sheath into which the viewing system and electrode are fitted, and movable member fixing the electrode at the rear end, the electrode fixing member formed of conductive material to fix the electrode at the rear end is internally provided within the movable member formed of insulating material, and is provided with the slit to form the flexible piece, the electrode holding space is formed within this slit, and both the clamping member pressing against the flexible piece on the side face to narrow the width of the electrode holding space, and the plug connecting part to which the plug at the tip of the electrode lead is to be connected are fitted to the faces of the movable member.

Now, in the embodiment formed as described above, in order to grip the electrode 19 firmly at the rear end by the electrode fixing structure, first of all the grip 34 of the clamping member 23 is loosened to release the pressing force applied to the flexible piece 22a of the electrode fixing member 22 and to allow the width of the slit 21 to expand due to the resilience of the flexible piece 22a, and then the rear of the electrode 19 is inserted into the electrode holding space 28 formed in the slit 21 of the electrode fixing member 22. When a predetermined length of the electrode 19 is inserted into the electrode holding space 28 and the grip 34 of the clamping member 23 rotated, the press rod 32 will be advanced, guided by the guide tube 31, and the pressed surface 25 of the flexible piece 22a of the electrode fixing member 22 will be pressed by the tip of the press-rod 32 to hold and fix the rear of the electrode 19 in the electrode holding space 28. At this time, as the electrode holding space 28 is formed to be of a cross-sectional shape substantially identical with that of the electrode 19, the electrode 19 will be held on the peripheral surface of its rear end, uniformly and strongly, in the electrode holding space 28. As soon as the electrode 19 is held in the electrode holding space 28, the electrode 19 will be electrically connected with the electrode lead so that a high frequency current may be fed to the tip of the electrode 19. Further, as the connecting mouthpiece 39 is pressed into the engaging hole 35 of the electrode fixing member 22, this connecting mouthpiece 39 prevents any forward or rearward movement or rotation of the electrode fixing member 22.

When in this state, if the resectoscope 1 is held by gripping the finger grip of the resectoscope sheath body 16 and the finger trigger 12 provided on the movable member 11, and force is applied to make the finger trigger 12 approach the other finger grip, the movable member 11 will slide forward and the current-carrying electrode 19 will project from the tip of the insulated part 18a at the end of the outer tube 18.

Therefore, when this projected electrode tip 19a is placed in contact with an affected part or the like, such part will be able to be resected.

After the resection is completed, if the force applied to the finger trigger 12 is weakened, the resilience of the plate spring 14 will cause the movable member 11 on which the finger trigger 12 is provided to slide rearwardly and the electrode tip 19a will retract into the outer tube tip part 18a.

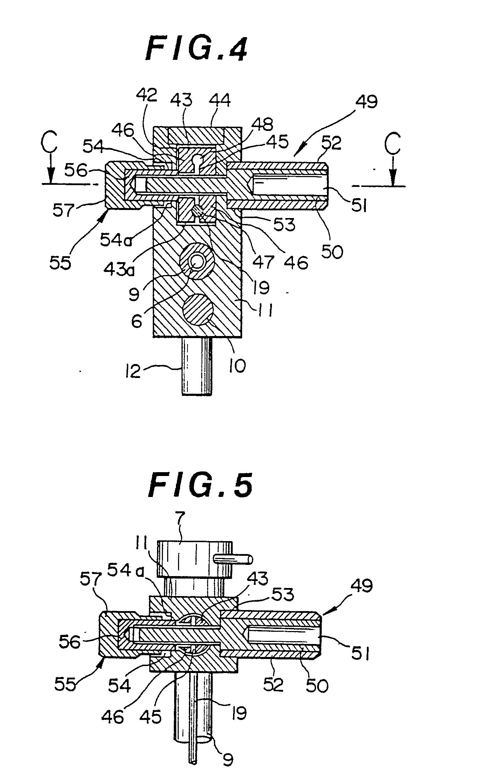

In the second exemplary embodiment, shown in Figures 4 and 5, a cylindrical space 42 is formed in the movable member 11 with its axial direction at right angles to the axial direction of the current-carrying electrode 19. An opening for inserting an electrode fixing member 43 is formed at the upper end of this space 42.

The electrode fixing member 43 arranged within the space 42 is formed of conductive material with a shape substantially identical to that of the space 42, but slightly shorter in the axial direction, and is arranged within the space 42, which is closed by a lid 44 formed of insulating material.

A slit 45 formed in this electrode fixing member 43 to coincide with the required direction of the current-carrying electrode 19 and opens on the circular bottom surface 43a of the electrode fixing member 43 and extend along the diameter of the circle. This slit 45 is formed to be of appropriate depth in the axial direction of the electrode fixing member 43, to form a flexible piece 46. Near the outer periphery at which this slit 45 opens, an electrode holding space 47 of circular cross-section having a diameter substantially identical to that of the rear end of the electrode 19 is formed from the front to the rear of the slit 45. Further, at the deepest part of this slit 45, an expanded bore 48 of substantially circular cross-section is formed from the front to the rear of the slit 45 to give a resiliency to the above mentioned flexible piece 46.

The electrode lead connecting part 49 is formed of a connecting mouthpiece 50, a plug inserting hole 51 opening on the rear end face of this connecting mouthpiece 50 and provided coaxially from the rear face toward the front, to receive the plug at the tip of the electrode lead, and an insulating member 52 is pressed at one large diameter end slightly into the movable member 11 and formed at the other end to be flush with the rear face of the connecting mouthpiece 50. The connecting mouthpiece 50 is inserted at the tip through a fitting opening 53 formed on the side face of the movable member 11, is passed through the electrode fixing member 43 in electrical contact therewith, and projects at the tip slightly out of an opening 54 formed on the opposite side face of the movable member 11, where a male screw-thread is provided on the outer periphery of the tip. The connecting mouthpiece 50 passes through the electrode fixing member 43 to prevent it from moving forward and rearward and rotating.

The clamping member 55 made to press against the side face of the flexible piece 46 of the electrode fixing member 43 to narrow the width of the slit 45 is fitted in an opening 54 for the projecting tip of the connecting mouthpiece.

This clamping member 55 is formed of a hollow press-rod 56 having a female screw-thread provided in the axial direction, to be screwed onto the male screw-thread on the tip of the connecting mouthpiece 50, and a control grip 57 of insulating material is secured to the rear of this press-rod 56 so that, when this grip 57 is rotated, the press-rod 56 is guided by the tip of the connecting mouthpiece 50 of the electrode lead connecting part 49 will advance into the movable member 11 through the clearance between the side face of the tip of the connecting mouthpiece 50 and the inner periphery of the opening 54, and its tip will press against one side of the flexible piece 46 of the electrode fixing member 43, to narrow the width of the slit 45. Further, the opening 54 has a projecting step inside, to reduce the clearance between the outer periphery of the pressing tube 56 and the opening 54, to prevent electrical leakage.

Now, with the second exemplary embodiment, formed as described above, in order to support the electrode 19 at the rear with the above mentioned electrode fixing structure, first of all, the control grip 57 of the clamping member 55 is turned to release the pressure applied to the electrode fixing member 43, and allow the width of the slit 45 to expand due to the resilience of the flexible piece 46 and then the rear of the electrode 19 is inserted into the electrode holding space 47 formed in the electrode fixing member 43. When a predetermined length of the electrode 19 is inserted into this electrode holding space 47 and the control grip 57 of the clamping member 55 is rotated to screw up the press-rod 56 on the tip of the connecting mouthpiece 50 against one face of the flexible piece 46 of the electrode fixing member 43, the electrode 19 will be held in the electrode holding space 47. At this time, as the electrode holding space 47 is formed to be of a cross-sectional shape substantially identical with that of the electrode 19, the electrode 19 will be held around its periphery uniformly and strongly, in the electrode holding space 47. As soon as the electrode 19 is held in the electrode holding space 47, the electrode 19 will be electrically connected with the electrode lead so that a high frequency current may be fed to the tip of the electrode 19.

As the connecting mouthpiece 50 passes through the middle of the electrode fixing member 43, this prevents the electrode fixing member 43 from moving forward or rearward, or rotating. Therefore, the electrode fixing member 43 is fixed in a simple manner, without requiring any other fixing means.

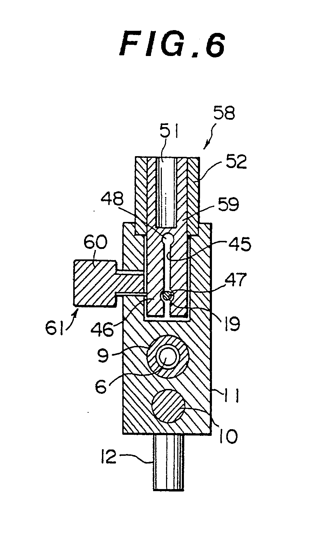

In the third exemplary embodiment, shown in Figure 6, an electrode fixing member 59 is of cylindrical form so that its axis may be in the direction at right angles to that of the current-carrying electrode 19. An electrode lead connecting part 58 is integrally formed at one end of this electrode fixing member 59. A screw-threaded hole is formed in the movable member 11 on the outer periphery to one side of the electrode fixing member 59. An insulated clamping member 60 is screwed into this screw-threaded hole to project and form a press-rod 61 making it possible to press the flexible piece 46 formed due to the provision of the slit 45. Otherwise, the third exemplary embodiment is substantially similar to the second embodiment, and like components bear the same reference numerals.

In embodiments of the present invention, the detailed structure of the resectoscope, and in particular the structure for advancing and retracting the finger trigger and electrode may be formed in various ways, providing the appropriate requirements are satisfied. The electrode tip can be properly positioned by setting the length from the tip of the electrode holding space formed in the slit of the electrode fixing member.

It is apparent that, in the first exemplary embodiment shown, for example, in Figure 1, the clamping member 23 can be fitted to the opposite side face of the movable member 11.

Further, for example, in Figure 2, the slit 21 formed in the electrode fixing member 22 can be formed in the horizontal direction (at right angles to the direction shown in Figure 2), the clamping member 23 can be fitted to the upper face of the movable member 11, and the plug connecting part 24 can be fitted to the right or left side face. In such cases, if the part holding the electrode 19 is more remote from the viewing system 6, the width of the movable member 11 may be made somewhat larger, and the position providing space for the electrode fixing member 22 may be displaced.

The present invention includes such modified embodiments where the shape of the movable member 11, normally of a rectangular cross-section, is made different, the fitting directions of the clamping member 23 or 55 and/or the plug connecting part 24 or 49 may be different.

Also, in the second exemplary embodiment shown, for example, in Figure 4, the construction may be such that a slit is formed in the small diameter part of the connecting mouthpiece 50, as in the first embodiment, so that when the movable member 11 and internally provided electrode fixing member 43 are pressed into a communicating hole, the plug connecting part 49 can be well fixed and will be positively connected electrically to the electrode fixing member.

As described above, according to the present invention, there are advantages that, as the electrode is electrically connected to the above mentioned electrode lead and is uniformly held about the entire periphery by the slit 21 or 45 of the fixing member 22 or 43, the side face of the rear end of the electrode 19 and the tip of the clamping member 23 or 55 will not be damaged or worn, even when used for a long period, and efficient electric contact to the electrode lead will be maintained, enabling a good flow of a high frequency current to be supplied, and no special step of aligning the clamping member with the current feeding mouthpiece or the electrode is required in the production process, so that assembling is made easy and further, as the part electrically connecting the electrode 19 with the electrode lead is encased within an insulating member, the device can be operated securely.

It will be apparent that different working modes can be formed in a wide range without deviating from the scope of the present invention as defined in the appended claims. The supply of current may be effected in various ways, and will normally be determined by the actual resection operation that is to be performed. In the simplest case, as has been illustrated, the return path for current may be via the body tissue and an appropriate electrode, or via the body tissue and the outer tube of the insertion sheath, which extends into the body cavity, and may be connected to a point of earth potential. In this case an insulating sleeve or coating (not shown) may be provided on a rigid rod electrode, extending from the front face of the electrode fixing member to a point adjacent the electrode tip within the insulated tip 18a of the insertion sheath. A direct electrical connection to the insertion sheath from the electrode tip may be provided, if the arrangement is resilient enough to avoid the sliding action of the insulated movable member 11. Finally, if required the electrode 19 may be in the form of a coaxial, or parallel pair of conductor members, if the plug connecting part and electrode fixing member are adapted to provide two connection paths, one for supply and one for return of the current flow.

The appropriate modifications required will be readily apparent to those skilled in the art.

## Claims

1. A resectoscope comprising an optical viewing system (2), an axially movable current-carrying electrode (19), an insertion sheath (4) within which the viewing system and electrode are inserted, an axially movable member (11) fixing the electrode at its rear end for controlling its axial movement, said movable member comprising an electrically insulating portion provided with an axial bore (20), an electrode fixing member (22) mounted to the movable member for fixing the rear end of the electrode, said electrode fixing member comprising a slit (21) to form a flexible piece and an electrode holding space within this slit, a clamping member (23) for radially pressing the flexible piece to narrow the width of the electrode holding space, and a plug connecting part (24) to which a plug at the tip of an electrode lead can be connected for electrically connecting the electrode and electrode lead together, said clamping member and plug connecting part being fitted to the lateral surface of the movable member, characterised in that the movable member (11) is made of an electrically insulating material, in that the electrode fixing member (22) is fitted in said axial bore (20), the clamping member (23) and the plug connecting part (24) being fitted to different side faces of the movable member (11), and in that the plug connecting part (24) is in direct electrical contact with the electrode fixing member (22).

2. A resectoscope according to Claim 1, characterised in that said plug connecting part (24) is fixed by a mouth piece inserted in an engaging hole of requisite depth in the electrode fixing member provided internally at the tip of the movable member.

3. A resectoscope according to Claim 1, characterised in that said plug connecting part (24) is fitted to the side part of the movable member by the mouthpiece, whose tip is passed through holes in the movable member and the electrode fixing member provided within said movable member.

4. A resectoscope according to Claim 1, characterised in that said plug connecting part is formed integrally with the electrode fixing member (59) provided within the movable member.

5. A resectoscope according to Claim 1, characterised in that said clamping member is formed of an insulated control grip (34) fitted with a press rod (32) advanced and retracted by screwing in an opening facing the pressed surface of the electrode fixing member.

6. A resectoscope according to Claim 1, characterised in that said clamping member has a hollow press-rod (56) fitted so as to be advanced and retracted by screwing or the like on the outer periphery of the tip of the mouthpiece, and it fitted with an insulated control grip (55).

## Patentansprüche

1. Resektoskop mit einem optischen Betrachtungssystem (2), einer axial beweglichen stromführenden Elektrode (19), einer Einführungsschutzhülle (4), in die das Betrachtungssystem und die Elektrode eingeführt sind, ein axial bewegliches Teil (11), das die Elektrode am rückwärtigen Ende hält und ihre axiale Bewegung steuert, wobei dieses bewegliche Teil einen elektrisch isolierenden Bereich aufweist, der mit einer axialen Bohrung (20) versehen ist, einen die Elektrode haltenden Teil (22), das mit dem beweglichen Teil zur Fixierung des rückwärtigen Endes der Elektrode verbunden ist, einen Schlitz (21) zur Bildung eines flexiblen Stückes und einen die Elektrode an diesem Schlitz haltenden Raum, mit einem Klemmteil (23) zur radialen Pressung des flexiblen Stückes zur Verringerung der Weite des die Elektrode haltenden Raumes und mit einem Steckkontakt (24) an den ein Stecker an der Spitze eines Elektrodenkabels anschließbar ist, zur elektrischen Verbindung der Elektrode und des Elektrodenkabels, wobei das Klemmteil und das Steckerverbindungsteil an der seitlichen Oberfläche des beweglichen Teils befestigt ist, dadurch gekennzeichnet, daß das bewegliche Teil (11) aus einem elektrisch isolierenden Material besteht, daß das die Elektrode haltende Teil (22) in der axialen Bohrung (20) angeordnet ist, daß das Klemmteil (23) und der Steckkontakt (24) an unterschiedlichen Seiten des beweglichen Teils (11) angeordnet sind und daß der Steckkontakt (24) in direktem elektrischen Kontakt mit dem die Elektrode haltenden Teil (22) steht.

2. Resektoskop nach Anspruch 1, dadurch gekennzeichnet, daß der Steckkontakt (24) durch ein Mundstück gehalten ist, das in ein Aufnahmeloch entsprechender Tiefe in dem die Elektrode haltenden Teil eingesetzt ist und das sich innen an der Spitze des beweglichen Teils befindet.

3. Resektoskop nach Anspruch 1, dadurch gekennzeichnet, daß der Steckkontakt (24) mittels des Mundstückes am Seitenteil des beweglichen Teiles befestigt ist, dessen Spitze durch Löcher in dem beweglichen Teil und dem die Elektrode haltenden Teil hindurchgeht, das sich in dem beweglichen Teil befindet.

4. Resektoskop nach Anspruch 1, dadurch gekennzeichnet, daß der Steckkontakt einstückig mit dem die Elektrode haltenden Teil (59), das in dem beweglichen Teil vorgesehen ist, ausgebildet ist.

5. Resektoskop nach Anspruch 1, dadurch gekennzeichnet, daß das Klemmteil aus einem isolierten Steuergriff (34) gebildet ist, mit einem Druckstab (32), der durch Schrauben in einer Öffnung vor- und zurückbewegbar ist, die der mit Druck beaufschlagten Fläche des die Elektrode haltenden Teiles zugewandt ist.

6. Resektoskop nach Anspruch 1, dadurch gekennzeichnet, daß das Klemmteil einen hohlen Druckstab (56) aufweist, der durch Schrauben od.dgl. vor- und zurückbewegbar ist und der an der äußeren Peripherie der Spitze des Mundstückes befestigt und mit einem isolierenden Steuergriff (55) versehen ist.

## Revendications

1. Résectoscope comprenant un système de visualisation optique (2), une électrode (19) de transmission du courant mobile axialement, un fourreau d'insertion (4) à l'intérieur duquel sont insérés le système de visualisation et l'électrode, un organe (11) mobile axialement qui fixe l'électrode à son extrémité arrière pour commander son mouvement axial, ledit mouvement dudit organe mobile comprenant une portion électriquement isolante munie d'un alésage axial (20), un organe (22) de fixation d'électrode monté sur l'organe mobile pour fixer l'extrémité arrière de l'électrode, ledit organe de fixation d'électrode comprenant une fente (21) pour former un élément flexible et un espace de retenue d'électrode dans cette fente, un organe de serrage (23) pour presser radialement l'élément flexible et rétrécir la largeur de l'espace de retenue d'électrode, et une pièce de liaison de fiche (24) à laquelle peut être reliée une fiche à l'extrémité d'un conducteur d'électrode pour relier électriquement l'électrode et le conducteur d'électrode l'un à l'autre, ledit organe de serrage et ladite pièce de liaison de fiche étant fixés à la surface latérale de l'organe mobile, caractérisé en ce que l'organe mobile (11) est réalisé en un matériau électriquement isolant, en ce que l'organe de fixation d'électrode (22) est disposé dans ledit alésage axial (20), l'organe de serrage (23) et la pièce de liaison de fiche (24) étant fixés sur les faces latérales différentes de l'organe mobile (11), et en ce que la pièce de liaison de fiche (24) est en contact électrique direct avec l'organe de fixation d'électrode (22).

2. Résectoscope selon la revendication 1, caractérisé en ce que ladite pièce de liaison de fiche (24) est fixée par une pièce d'embouchure insérée dans un trou d'engagement de profondeur appropriée dans l'organe de fixation d'électrode prévu à l'intérieur de la pointe de l'organe mobile.

3. Résectoscope selon la revendication 1, caractérisé en ce que ladite pièce de liaison de fiche (24) est fixée à la partie latérale de l'organe mobile par l'embouchure, dont la pointe traverse des trous de l'organe mobile et de l'organe de fixation d'électrode prévu à l'intérieur dudit organe mobile.

4. Résectoscope selon la revendication 1, caractérisé en ce que ladite pièce de liaison de fiche est formée d'un seul tenant avec l'organe de fixation d'électrode (59) prévu à l'intérieur de l'organe mobile.

5. Résectoscope selon la revendication 1, caractérisé en ce que ledit organe de serrage est formé par une poignée de commande isolée (34) munie d'une tige de pression (32) qui est avancée et rétractée par vissage dans une ouverture faisant face à la surface pressée de l'organe de fixation d'électrode.

6. Résectoscope selon la revendication 1, caractérisé en ce que ledit organe de serrage comprend une tige de pression creuse (56) fixée de manière à être avancée et rétractée par vissage ou analogue sur la périphérie extérieure de la pointe de l'embouchure, et est muni d'une poignée de commande isolée (55).

*FIG.1*

*FIG.2*

*FIG.3*

0 084 419

# FIG.4

# FIG.5

# FIG.6